# EUROPEAN PATENT SPECIFICATION

(11) **EP 2 234 975 B1**
(45) Date of publication and mention of the grant of the patent: **25.04.2012**
(21) Application number: 08707041.3
(22) Date of filing: 15.01.2008
(51) Int. Cl.: C07D 211/60

(54) **Process for producing pipecolic-2-acid-2 ',6'-xylidide useful as an intermediate for the preparation of local anesthetics**
Verfahren zur Herstellung von Pipecol-2-säure-2',6'-xylidid als Zwischenprodukt für die Herstellung von Lokalanästhetika
Procédé de production d'acide-2-pipécolique-2',6'-xylidide utile en tant qu'intermédiaire pour la préparation d'anesthésiques locaux

(43) Date of publication of application: 06.10.2010
(73) Proprietor: Pharmathen S.A., 15351 Pallini Attikis (GR)
(72) Inventor: SONI, Rohit, Ravikant, GR-551 34 Kalamaria Thessaloniki (GR); KOFTIS, Theocharis, GR-542 48 Thessaloniki (GR); GEORGOPOULOU, Ionna, GR-546 29 Anatoli A Thessaloniki (GR); KARAGIANNIDOU, Evrykleia, GR-566 26 Sykies Thessaloniki (GR)
(86) International application number: PCT/EP2008/000237
(87) International publication number: WO 2009/089842

(56) References cited:
- WO-A-96/12700
- GB-A- 824 542
- ACS M ET AL: "Study of the Diastereoisomers Formed between (N-alkyl)-pipecolic acid-anilides and 2R,3R-tartaric acid or O,O ?-dibenzoyl-2R,3R-tarta ric acid. Do the Tartaric Acids Form Molecular-Complexes, instead of Salts During Optical Resolutions?" TETRAHEDRON, ELSEVIER SCIENCE PUBLISHERS, AMSTERDAM, NL, vol. 52, no. 5, 29 January 1996 (1996-01-29), pages 1637-1642, XP004104468 ISSN: 0040-4020

## Description

### TECHNICAL FIELD OF THE INVENTION

The present invention relates to an improved process for the preparation of pipecolic-2-acid-2',6'-xylidide compounds or derivatives thereof and in particular to a method suitable for the manufacture of an optically active form thereof, which represents an important intermediate for the preparation of local anesthetics, such as Ropivacaine or Levobupivacaine or salts thereof.

### BACKGROUND OF THE INVENTION

Pipecolic-2-acid-2',6'-xylidide, particularly in the optically active form thereof, is useful as an intermediate in the preparation of local anesthetics. For example L-pipecolic-2-acid-2',6'-xylidide of formula 1 is employed as an intermediate in the manufacturing of local anesthetics such as Levobupivacine and Ropivacaine.

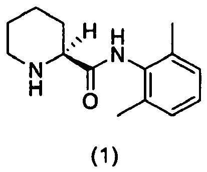

Various methods are already known for the preparation of L-pipecolic-2-acid-2',6'-xylidide in an optically active form. However, the prior art has encountered substantial difficulties in the preparation of L-pipecolic-2-acid-2',6'-xylidide or salt thereof due to formation of undesired by-products.

WO-A-85/00599 discloses the synthesis of L-pipecolic-2-acid-2',6'-xylidide which comprises resolution of 2-pipecolic acid and its conversion to L-2-pipecolic acid chloride hydrochloride with PCl₅ in acetyl chloride as a solvent which on further condensation with 2,6-xylidine gives the corresponding L-pipecolic-2-acid-2',6'-xylidide. The main drawbacks of this route lie in the handling of acetyl chloride as a solvent on plant scale and also the filtration of the pipecolic acid chloride hydrochloride which is an intermediate in the process. Further, the use of PCl₅ on a large scale is problematic, as PCl₅ is liable to react with atmospheric moisture; also, waste separation from the acid chloride intermediate is difficult. Moreover, the phosphate waste streams which are generated are difficult to treat or discard otherwise.

WO-A-96/12700 discloses a process for preparing Levobupivacine, (±)-Bupivacaine and other N-alkyl analogues, wherein 2-pipecolic acid hydrochloride is chlorinated with thionyl chloride or oxalyl chloride to 2-pipecolic acid chloride hydrochloride, and on further amidation with 2,6-dimethyl aniline without isolation gives the resultant pipecolic-2-acid-2',6'-xylidide derivatives. However, the handling of thionyl chloride in an industrial scale is difficult, and the reaction generates harmful gases like sulfur dioxide posing environmental problems.

EP-B-0784608 discloses a process for preparing Levobupivacaine and Ropivacaine, from natural and inexpensive L-Lysine, using a chirality pool approach to get the single S-enantiomer, However, this process comprises a long sequence of reactions comprising protection and deprotection of α-amino group and involving hazardous diazotization reaction, as well.

GB 824542 discloses the preparation of N-alkyl-pipecolic-2-acid-2',6'-xylidide starting from 2-picolinic acid by its reaction with suitable aromatic amine with POCl₃; the resultant Picolinic-2-acid-2',6'-xylidide is then reduced to the corresponding Pipecolic-2-acid-2',6'-xylidide using PtO₂ as a catalyst. The disadvantage of this process is the use of very costly Pt metal catalyst.

Accordingly, there still exists a need for an efficient, cost-effective and safer process without hazardous undesired by-products.

### SUMMARY OF THE INVENTION

It is, therefore, an object of the present invention to provide an improved process for the preparation of pipecolic-2-acid-2',6'-xylidide or derivative thereof, which overcomes the deficiencies of the prior art.

Another object of the present invention is to provide an environmental friendly method of preparing pipecolic-2-acid-2',6'-xylidide in an optically active form or derivative thereof having high chiral purity, which is efficient and results to reduction of the cost of production.

In accordance with the above objects of the present invention, a process for the preparation of pipecolic-2-acid-2',6'-xylidide or derivative thereof is provided comprising hydrogenation of picolinic-2-acid-2',6'-xylidide in the presence of a Raney-nickel as a catalyst. Preferred embodiments of the present invention are set out in dependent claims 2 to 9. Other objects and advantages of the present invention will become apparent to those skilled in the art in view of the following detailed description.

### DETAILED DESCRIPTION OF THE INVENTION

The present invention relates to an improved process for the manufacture of the compound pipecolic-2-acid-2',6'-xylidide or derivatives thereof.

According to the present invention, the process for the preparation of L-pipecolic-2-acid-2',6'-xylidide comprises the following steps:
- A starting material 2-picolinic acid hydrochloride salt is suspended in dichloromethane and reacted with oxalyl chloride to get the corresponding acid chloride;
- The *in situ* generated acid chloride is then condensed with 2,6-xylidine to get the picolinic-2-acid-2',6'-xylidide derivative, which on further hydrogenation with Raney Nickel as a catalyst provides pipecolic-2-acid-2',6'-xylidide;
- Racemic pipecolic-2-acid-2',6'-xylidide is then resolved to the L-isomer with Dibenzoyl-L-tartaric acid to obtain chiral purity in the range of 90-95%.
- Further enhancement of the chiral purity up to ≥99.5% is achieved by crystallization of the acid addition salts thereof with alkanoic acid in non-ketonic and non-alcoholic solvent in non-aqueous medium.

According to the present invention, pipecolic-2-acid-2',6'-xylidide or derivative thereof is prepared from the commercially available and less expensive 2-picolinic acid, which is used as the key starting material. The HCL salt thereof (i.e. 2-picolinic acid hydrochloride salt) prepared *in situ* is reacted with oxalyl chloride to generate the corresponding acid chloride avoiding phosphorus (PCl₅, PCl₃ and POCl₃.) and sulphurous (SOCl₂ and SO₂Cl₂) chlorinating agents and the use of acetyl chloride.

The acid chloride is then condensed with 2,6-xylidine at -5 to 0°C in presence of an organic base to obtain the corresponding Picolinic-2-acid-2',6'-xylidide.
The various organic bases such as pyridine or molar excess of the 2,6-xylidine or trialkylamines such as triethylamine, N-ethyl-N,N-diisopropylamine etc. can be employed; the most preferred organic base is pyridine.

The resultant picolinic-2-acid-2',6'-xylidide is then converted to pipecolic-2-acid-2',6'-xylidide by selective hydrogenation of the pyridine ring to piperidine ring in the presence of the carbocyclic aromatic ring. Raney-Ni was chosen among other reducing reagents as the less expensive. The use of PtO₂ 5% (acetic acid-methanol, 3.5 bar, 50°C) has been proven to be efficient, however it is expensive.

The reduction conditions has been tested and proved crucial, regarding all possible parameters (i.e. solvent, temperature, pressure, time).
When the reduction was performed in methanol at 90°C under 75 psi hydrogen pressure using Raney-Ni, after 24 hours the product 3 was found at 73 %, together with 16 % of starting compound 2 and 8 % of the methylated analogue 4 (R = Me) (yields are according to HPLC results) [see Table 1, entry 1]. When the hydrogen pressure was increased [see Table 1, entries 2-4], the starting compound 2 reduced below 1 %, but the methylated product 4 increased as by-product of the reduction. In order to eliminate this formation, water was replaced as solvent and acetic acid was added to increase the solubility of compound 2. That assumption proved correct, but total 24.5 % of unknown impurities observed, probably due to acetylation of nitrogen [see Table 1, entry 5]. Using only acetic acid as solvent the reduction was very slow [only 35 % conversion after 48 h, see Table 1, entry 6]. The best results came after changing back to methanol and buffering the system with acetic acid in order to moderate the basicity of the solution [see Table 1, entries 8-10]. It was found that by keeping the amount of the acetic acid as low as 0.5 ml/g of starting compound 2, both starting compound 2 and byproduct 4 (R = Me) were detected in desirable amounts [see Table 1, entry 10]. 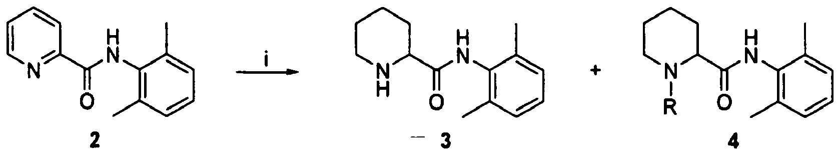 i. Hydogenation conditions for the reduction of 2,6-picoloxylidide.

**TABLE 1**

| **S/N** | **Raney-Ni (% w/w)** | **Pressure (psi)** | **Solvent** | **Temp. (° C)** | **Reaction Time (h)** | **2 (%)** | **3 (%)** | **4^{a} (%)** | **Other (%)** |
|---|---|---|---|---|---|---|---|---|---|
| 1. | 50 | 75 | MeOH | 90 | 24 | 16 | 73 | 8 | 3 |
| 2. | 30 | 200 | MeOH | 90 | 24 | 0.3 | 72 | 25 | 3 |
| 3. | 50 | 200 | MeOH | 90 | 24 | 0.5 | 83 | 13.5 | 2 |
| 4. | 50 | 260 | MeOH | 90 | 24 | 0.02 | 80.5 | 14 | 5.5 |
| 5. | 60 | 260 | H₂O - AcOH (15/1) | 50 | 48 | 0.5 | 75 | 0 | 24.5 |
| 6. | 60 | 260 | AcOH | 50 | 48 | 61.8 | 35 | 0 | 3 |
| 7. | 60 | 260 | nPrOH - AcOH (10/1) | 50 | 28 | 12.6 | 86 | 0.51 | 1.49 |
| 8. | 60 | 260 | MeOH/AcOH (10/1) 10 volumes | 50 | 28 | 0.1 | 98.5 | 0.05 | 1.35 |
| 9. | 60 | 260 | MeOH/AcOH (5/1) 10 volumes | 50 | 28 | 0.5 | 98.5 | 0.07 | 0.93 |
| 10. | 60 | 260 | MeOH/AcOH (9/1) 5 volumes | 50 | 28 | 0.3 | 98.7 | 0.06 | 0.94 |

| | | | | | | | | | |
|---|---|---|---|---|---|---|---|---|---|
| *a*: R = Methyl, except entry *no. 7*, where is *n*-propyl.. | | | | | | | | | |

Finally, when *n*-Propanol was used as solvent together with acetic acid, the results were poor [see Table 1, entry 7].
Overall, by keeping a hydrogen pressure at 260 psi, using 60 % w/w of Raney-Ni at 50°C for 28 hours, in a solvent system of methanol - acetic acid 9:1 in concentration as low as 5 volumes, the product in reaction mixture was identified by HPLC as 98.5 % that after work up of the crude mixture and crystallization afforded the desired product in more than 99.5 % purity and 85 % yield.

The resultant racemic pipecolic-2-acid-2',6'-xylidide is then resolved to the L-isomer using dibenzoyl-L-tartaric acid in isopropanol to achieve the chiral purity in the range of 90-95% according to the process disclosed in *"*Acta scandinavica " 1987 B41 759-61*.*

Further enhancement of the chiral purity at least 99.5% is achieved by crystallization of the acid addition salts thereof with alkanoic acid in non-ketonic and non-alcoholic solvent in non-aqueous medium. The most preferred alkanoic acids are acetic acid, propionic acid, butanoic acid and trifluoroacetic acid.

The process of the present invention will be demonstrated in more details with reference to the following examples, which are provided by way of illustration only and should not be construed as limit to the scope of the reaction in any manner.

### Example 1: Preparation of Picolinic-2-acid-2',6-'ylidide

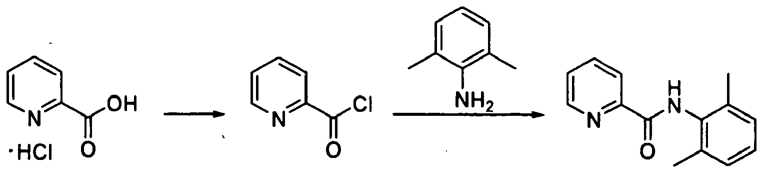

A three neck round bottom flask equipped with mechanical stirrer, dropping funnel and condenser, under argon atmosphere was charged with 19.4 g. of 2-picolinic acid hydrochloride, 0.3 ml of *N*,*N-*dimethylformamide and 225 ml of dichloromethane. Then, a solution of 11 ml of oxalyl chloride (1.05 equiv) in 4 ml of dichloromethane was added to the mixtre in a period of 30 min at 20-25°C. The reaction mixture was kept at 20-25° C with vigorous stirring for additional 4.5 hours and then cooled to -5° C. A solution of 15 ml of 2,6-xylidine (1.0 equiv) and 20 ml of pyridine (2.0 equiv) in 15 ml dichloromethane was added in a period of 1 hour at the same temperature. The reaction mixture was stirred for 1 hour at -5 to 0°C, warmed to 20° C and maintained for an additional 1 hour for the completion of the reaction. To the reaction mass 200 ml of water were added, stirred for additional 15 min and the mixture was transferred to a separatory funnel.

The organic layer was separated and washed with 100 ml of water. The aqueous layers were combined and extracted once with 50 ml of dichloromethane. The two organic layers were combined, dried over MgSO₄, filtered and the solvents were removed in a rotary evaporator.

The crude solid mass obtained (∼32g.) was recrystallized with 75 ml of cyclohexane, filtered and the crystals were washed with 15 ml of cyclohexane to provide after drying 26.0 g. of slightly yellowish crystals of pipecolic-2-acid-2',6'-xylidide in ∼94 % yield, M.P.104.2- 104.5°C. ¹H NMR (CDCl₃, 300 MHz) : δ = 9.49 (s, 1H), 8.59 (dd, *J*= 5.0, 0.8 Hz, 1H), 8.27 (dd, *J*= 7.8, 0.8 Hz, 1H), 7.84 (dt, *J*= 7.8, 1.5 Hz, 1H), 7.42 (ddd, *J*= 7.8, 5.0, 1.5 Hz, 1H), 7.11 (m, 3H), 2.28 (s, 6H) ppm; ¹³C NMR (CDCl₃, 75 MHz): δ = 162.1, 149.5, 147.9, 137.3, 135.1, 133.6, 127.9, 126.9, 126.2, 122.2, 18.3 ppm.

### Example-2 Preparation of Pipecolic-2-acid-2',6'-xylidide

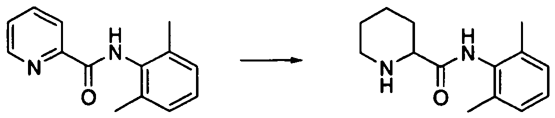

To an SS reaction bottle equipped with a mechanical stirrer, 25g of picolinic-2-acid-2',6'-xylidide, 625 ml of methanol, 63 ml of acetic acid and 15 g. of Raney nickel were added.

Then, in the mixture argon was passed through for 5 min, sealed and charged with hydrogen at 18 Kg/cm² (260 psi). The reaction mixture was heated at 50°C and maintained under the same pressure and temperature for 28-30 hours until completion of reaction. The reaction mass was filtered to remove the catalyst and mother liquor was concentrated in a rotary evaporator. The crude reaction mass was dissolved in 250 ml of toluene, transferred to a separatory funnel, 250 ml of water was added and basified with 50 ml of 20% aqueous sodium hydroxide.

The organic layer was separated and the aqueous layer was extracted twice with 100 ml of toluene. The combined organic layer after extraction was dried over MgSO₄ and concentrated in a rotary evaporator.

The crude solid mass obtained was recrystallized from 70 ml of toluene to provide 18.5 g of slightly pink solid of pipecolic-2-acid-2',6'-xylidide in ~72 % yield, M.P.115.7-116.2° C; ¹H NMR (CDCl₃, 300 MHz): δ = 8.31 (s, 1H), 7.00 (m, 3H), 3.32 (dd, *J*= 3.2, 9.7 Hz, 1H), 3.0 (m, 1H), 2.68 (m, 1H), 2.14 (s, 6H), 1.98 (m, 1H), 1.93 (s, 1H)1.77 (m, 1H), 1.59-1.50 (m, 2H), 1.47-1.37 (m, 2H) ppm; ¹³C NMR (CDCl₃, 75 MHz): δ = 172.1, 134.7, 133.4, 127.6, 126.5, 59.9, 45.3, 30.0, 25.6, 23.6, 18.1 ppm.

### Example-3 Preparation of L-Pipecolic-2-acid-2',6'-xylidide

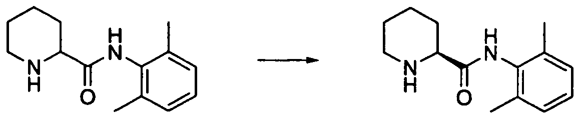

In a three neck round bottom flask equipped with thermometer, mechanical stirrer and condenser, 21 g. of dibenzoyl-L-tartatric acid monohydrate (0.52 equivalents) were charged together with 250 ml of isopropanol. The suspension was heated to reflux until clear solution. 25 g of pipecolic-2-acid-2',6'-xylidide (1.0 equiv) were charged at reflux temperature and allowed to cool slowly to 20°C during a period of 90 minutes. The precipitated salt was collected by filtration, washed with 50 ml isopropanol and transferred to a round bottom flask equipped with magnetic stirrer. 150 ml of ethyl acetate, 250 ml water and 13 ml of 50% a sodium hydroxide solution were added and the mixture was stirred vigorously for 10 min, before being transferred to a separating funnel. The organic layer was separated and the aqueous layer was extracted twice with 125 ml of ethyl acetate. The three organic extracts were combined, dried with MgSO₄, the solids were filtered off and the solvents removed in a rotary evaporator to provide 10 g. of L-pipecolic-2-acid-2',6'-xylidide in 40 % yield with chiral purity of 90-92% S-isomer.; The further enhancement of chiral purity was done by the following purification.

In a round bottom flask 10.0 g of L-pipecolic-2-acid-2',6'-xylidide (with 90-92% S-isomer from the previous step) together with 140 ml toluene were added and the suspension was heated to reflux until clear solution. The solution was cooled down to 60°C and 3.8 ml of acetic acid (1.5 equiv) was added. The precipitated salt was filtered and recrystallized with 164 ml of toluene to provide 6.5g of L-pipecolic-2-acid-2',6'-xylidide acetate salt that was transferred to a round bottom flask equipped with a magnetic stirrer. 100 ml of ethyl acetate, 100 ml water and 10 ml of aqueous NaOH (50 % w/w) were added and the mixture was stirred vigorously for 10 min, before being transferred to a separating funnel.

The organic layer was separated and the aqueous layer was extracted twice with 100 ml of ethyl acetate. The three organic extracts were combined, dried with MgSO₄, the solids were filtered off and the solvents were removed in a rotary evaporator to provide 6.0g of L-pipecolic-2-acid-2',6'-xylidide in 60 % yield with chiral purity ≥ 99.5%. [α] = +47.3 (c = 1.0, 1N HCl), ¹H NMR (CDCl₃, 300 MHz): δ = 8.31 (s, 1H), 7.00 (m, 3H), 3.32 (dd, *J*= 3.2, 9.7 Hz, 1H), 3.0 (m, 1H), 2.68 (m, 1H), 2.14 (s, 6H), 1.98 (m, 1H), 1.93 (s, 1H)1.77 (m, 1H), 1.59-1.50 (m, 2H), 1.47-1.37 (m, 2H) ppm; ¹³C NMR (CDCl₃, 75 MHz): δ = 172.1, 134.7, 133.4, 127.6, 126.5, 59.9, 45.3, 30.0, 25.6, 23.6, 18.1 ppm.

Therefore, the present invention provides a process wherein pipecolic-2-acid-2',6'-xylidide, or optically active form thereof such as L-pipecolic-2-acid-2',6'-xylidide, useful as an intermediate for the preparation of local anesthetics such as Ropivacaine, Levobupivacaine or salts thereof, can be prepared in an improved manner employing as a starting material picolinic-2-acid-2',6'-xylidide or derivative thereof.

## Claims

1. A process for the preparation of pipecolic-2-acid-2',6'-xylidide or derivative thereof wherein it comprises hydrogenation of picolinic-2-acid-2',6'-xylidide of formula 2 in the presence of a Raney-nickel as a catalyst.

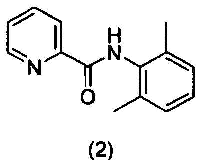

2. The process according to claim 1, wherein a solvent is used for the above reduction selected from alcohol and lower aliphatic acid.

3. The process according to claim 2, wherein said solvent is methanol and acetic acid.

4. The process according to claim 1, wherein the reduction is carried out at 40-80°C and a hydrogen pressure of 10-30 Kg/cm², preferably at 50°C and under 18 Kg/cm² hydrogen pressure.

5. The process according to claim 1, wherein it further comprises the following steps:
a) Reacting Picolinic-2-acid hydrochloride with oxalyl chloride to get the corresponding acid chloride;
b) Reacting the in situ generated acid chloride of step a) with 2,6-xylidine in the presence of an organic base to obtain the desired picolinic-2-acid-2',6'-xylidide.

6. The process according to claim 5, wherein said organic base is selected from a group consisting of pyridine derivative and trialkylamine.

7. The process according to claim 6, wherein said organic base is pyridine.

8. The process according to claim 5, wherein it further after hydrogenation the obtained racemic pipecolic-2-acid-2',6'-xylidide is resolved to the L-isomer with Dibenzoyl-L-tartaric acid to obtain L-pipecolic-2-acid-2',6'-xylidide of formula 1, having chiral purity in the range of 90-95%.

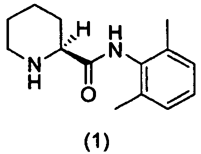

9. The process according to claim 8, wherein it further comprises crystallization of L-pipecolic-2-acid-2',6'-xylidide acid addition salt in a non-alcoholic and non-ketonic solvent in non-aqueous medium to obtain L-pipecolic-2-acid-2',6'-xylidide of chiral purity at least 99.5%.

## Patentansprüche

1. Ein Verfahren zur Herstellung von Pipecolinsäure-2-säure-2 ', 6'-xylidid oder ein Derivat davon, wobei es umfasst Hydrierung von Picolinsäure-2-Säure', 6'-xylidid der Formel 2 in Gegenwart eines Raney -Nickel als Katalysator.

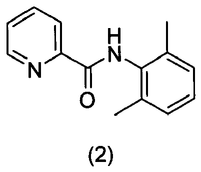

2. Das Verfahren nach Anspruch 1, wobei ein Lösungsmittel für die Reduktion von Alkohol und niederen aliphatischen Säure ausgewählt ist.

3. Das Verfahren nach Anspruch 2, **dadurch gekennzeichnet, dass** das Lösungsmittel Methanol und Essigsäure ist.

4. Das Verfahren nach Anspruch 1, wobei die Reduktion bei 40-80°C und einem Wasserstoffdruck von 10-30 kg / cm², vorzugsweise bei 50°C und unter 18 Kg / cm² Wasserstoffdruck durchgeführt ist.

5. Das Verfahren nach Anspruch 1, wobei es ferner die folgenden Schritte umfasst:
a) Reaktion Picolinsäure-2-Hydrochlorid mit Oxalylchlorid um den Säurechlorid zu erhalten;
b) Umsetzung des im Schritt a) in situ erhaltenden Säurechlorid mit 2, 6-Xylidin beim zufügen einer organischen Base oder Pyridin-Derivat, um Pipecolinsäure-2-säure-2 ',6'-xylidid zu erhalten.

6. Das Verfahren gemäß Anspruch 5, **dadurch gekennzeichnet dass** die organische Base von einer Gruppe aus Pyridin-Derivat und Trialkylamin ausgewählt ist.

7. Das Verfahren gemäß Anspruch 6, **dadurch gekennzeichnet dass** die organische Base Pyridin ist.

8. Das Verfahren nach Anspruch 5, wobei es ferner nach Hydrierung, der erhaltenen racemischen Pipecolinsäure-2-säure-2 ',6'-xylidid mit dem L-Isomer mit Dibenzoyl-L-Weinsäure gelöst ist, um L-Pipecolinsäure-2-, 6'-xylidid der Formel 1, mit chiralen Reinheit im Bereich von 90-95% zu erhalten.

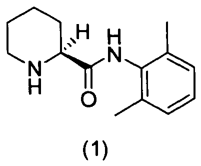

9. Das Verfahren nach Anspruch 8, wobei es ferner umfasst, Kristallisation von L-Pipecolinsäure-2-acid-2',6'-xylidid Säureadditionssalze in einer nicht-alkoholische und nicht-Keton Lösungsmittel in nicht-wässerigen Medium, um L-Pipecolinsäure-2-Säure-2 ', 6'-xylidid von chiralen Reinheit von mindestens 99,5% zu erhalten.

## Revendications

1. Un procédé pour la préparation de l'acide 2-pipécolique-2',6'-xylidide ou dérivé de celui-ci dans lequel est compris l'hydrogénation de l'acide-2-picolinique-2',6'-xylidide de formule 2 en présence de nickel de Raney comme catalyseur.

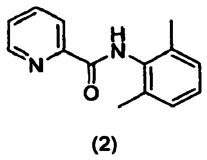

2. Le procédé selon la revendication 1, dans lequel un solvant est utilisé pour la réduction ci-dessus sélectionné parmi un alcool et un acide aliphatique inférieur.

3. Le procédé selon la revendication 2, dans lequel ledit solvant est le méthanol et l'acide acétique.

4. Le procédé selon la revendication 1, dans lequel la réduction est effectuée à 40-80°C et sous une pression d'hydrogène de 10-30 kg/cm², préférablement à 50°C et sous une pression d'hydrogène de 18 kg/cm².

5. Le procédé selon la revendication 1, dans lequel sont comprises en outre les étapes suivantes:
a) Réagir le chlorhydrate de l'acide 2-picolinique avec du chlorure d'oxalyle pour obtenir le chlorure d'acide correspondant;
b) Réagir le chlorure d'acide généré *in situ* à l'étape a) avec la 2,6-xylidine en présence d'une base organique pour obtenir l'acide 2-picolinique-2',6'-xylidide.

6. Le procédé selon la revendication 5, dans lequel ladite base organique est sélectionnée à partir du groupe consisté de dérivé de la pyridine et de trialkyl amine.

7. Le procédé selon la revendication 6, dans lequel ladite base organique est la pyridine.

8. Le procédé selon la revendication 5, dans lequel après hydrogénation, l'acide -2-pipécolique-2',6'-xylidide racémique est ensuite résolu (dédoublé) par l'acide dibenzoyle-L-tartrique pour obtenir l'acide L-2-pipécolique-2',6'-xylidide de formule 1, ayant une pureté optique (chirale) dans la gamme de 90-95%.

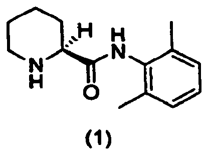

9. Le procédé selon la revendication 8, dans lequel est comprise en outre la cristallisation du sel d'addition acide de l'acide L-2-pipécolique-2',6'-xylidide dans un solvant non-alcoolisé et non-cétonique dans un milieu non-aqueux pour obtenir l'acide L-2-pipécolique-2',6'-xylidide d'une pureté optique (chirale) d'au moins 99.5%.
